(19) Europäisches Patentamt
European Patent Office
Office européen des brevets

(11) EP 4 185 291 B1

(12) **EUROPEAN PATENT SPECIFICATION**

(45) Date of publication and mention of the grant of the patent:
**09.07.2025 Bulletin 2025/28**

(21) Application number: **21755253.8**

(22) Date of filing: **23.07.2021**

(51) International Patent Classification (IPC):
***A61K 31/454** (2006.01)* ***A61P 25/28** (2006.01)*

(52) Cooperative Patent Classification (CPC):
**A61K 31/454; A61P 25/28**

(86) International application number:
**PCT/US2021/042877**

(87) International publication number:
**WO 2022/020663 (27.01.2022 Gazette 2022/04)**

(54) **LOW DOSE REGIMEN AND FORMULATION OF A 5-METHYL-1,2,4-OXADIAZOL-3-YL COMPOUND**

NIEDRIGDOSIERTES SCHEMA UND FORMULIERUNG EINER 5-METHYL-1,2,4-OXADIAZOL-3-YL-VERBINDUNG

SCHÉMA POSOLOGIQUE À FAIBLE DOSE ET FORMULATION D'UN COMPOSÉ 5-MÉTHYL-1,2,4-OXADIAZOL-3-YLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **23.07.2020 US 202063055362 P**

(43) Date of publication of application:
**31.05.2023 Bulletin 2023/22**

(73) Proprietor: **Eli Lilly and Company**
**Indianapolis, IN 46285 (US)**

(72) Inventors:
- **KIELBASA, William Brian**
**Indianapolis, Indiana 46206-6288 (US)**
- **MERGOTT, Dustin James**
**Indianapolis, Indiana 46206-6288 (US)**

(74) Representative: **Eli Lilly and Company Limited**
**8 Arlington Square West**
**Downshire Way**
**Bracknell Berkshire RG12 1PU (GB)**

(56) References cited:
**WO-A1-2018/140299 WO-A1-2020/028141**

- **KEVIN HAEHL: "The Importance of Convenient Dosing Formulations for Elderly Patients", PHARMA'S ALMANAC, 1 February 2016 (2016-02-01), pages 1 - 10, XP055852011, Retrieved from the Internet <URL:https://www.pharmasalmanac.com/articles/q1-unither-the-importance-of-convenient-dosing-formulations-for-elderly-patients> [retrieved on 20211017]**
- **PRETORIUS ET AL: "Reducing the Risk of Adverse Drug Events in Older Adults", AMERICAN FAMILY PHYSICIAN, 1 January 2013 (2013-01-01), United States, pages 331 - 336, XP055852012, Retrieved from the Internet <URL:https://www.aafp.org/afp/2013/0301/afp20130301p331.pdf> [retrieved on 20211017]**
- **BARTOLOMÉ-NEBREDA JOSE M. ET AL: "O-GlcNAcase inhibitors as potential therapeutics for the treatment of Alzheimer's disease and related tauopathies: analysis of the patent literature", EXPERT OPINION ON THERAPEUTIC PATENTS, 8 July 2021 (2021-07-08), GB, pages 1 - 38, XP055851969, ISSN: 1354-3776, DOI: 10.1080/13543776.2021.1947242**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 185 291 B1

## Description

The present invention relates to a low-dose regimen and low-dose formulation of the O-GlcNAcase (OGA) inhibitor N-[4-fluoro-5-[[(2S,4S)-2-methyl-4-[(5-methyl-1,2,4-oxadiazol-3-yl)methoxy]-1-piperidyl]methyl]thiazol-2-yl] acetamide, or pharmaceutically acceptable salt thereof, to treat neurodengerative diseases, including, neurodegenerative tauopathies such as Alzheimer's disease (AD), frontotemporal dementia (FTD), corticobasilar syndrome (CBS), and progressive supranuclear palsy (PSP).

U.S. Patent No. 10,081,625 discloses certain compounds, including 5-methy-1,2,4-oxadiazol-3-yl compounds, including N-[4-fluoro-5-[[(2S,4S)-2-methyl-4-[(5-methyl-1,2,4-oxadiazol-3-yl)methoxy]-1-piperidyl]methyl]thiazol-2-yl] acetamide which are OGA inhibitors useful for treating neurodegenerative diseases and disorders, such as AD and PSP.

Patients suffering from neurodegenerative diseases, including AD, PSP, and other neurodegenerative tauopathies, may develop difficulty swallowing as the disease progresses. Thus, it is desired that prescribed dosages of a drug are of a low dose providing a small unit dosage form, in order to faciliate swallowing when the drug is administered to the patient. Furthermore, in view of the progressive cognitive impairment often associated with neurodegenerative diseases such as AD, reducing the number of dosage administrations per day will help minimize the risk that a patient will miss a dose and will also reduce the burden on the caregiver responsible for ensuring proper and timely administration of the drug. In addition, lower doses and less frequent dose administration will ameliorate or eliminate potential side effects, particularly those that might arise with chronic adminstration (See W. Cook, et. al., 2020 Society of Toxicology Annual Meeting (virtual), Abstract No. 2911, May 12, 2020; https://www.toxicology.org/events/am/AM2020/docs/2020-ePoster-index.pdf).

Thus, low doses of OGA inhibitors are desired to provide treatment for neurodegenerative diseases, such as AD and PSP and other neurodegenerative tauopathies. In addition, administration of OGA inhibitors of no more than twice per day, and preferably once per day, are further desired for treatment of neurodegenerative diseases, including neurodegenerative tauopathies such as AD, FTD, CBS, and PSP.

Accordingly, in one embodiment, the invention provides a compound of Formula I:

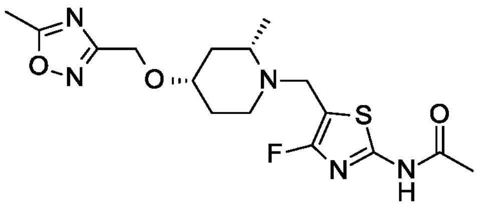

Formula I

,

or a pharmaceutically acceptable salt thereof, for use in treating a neurodegenerative disease wherein the compound, or pharmaceutically acceptable salt thereof is administered orally with a total dose of the compound of 0.1 mg/day to 5 mg/day.

In an embodiment, the invention provides a compound of Formula I:

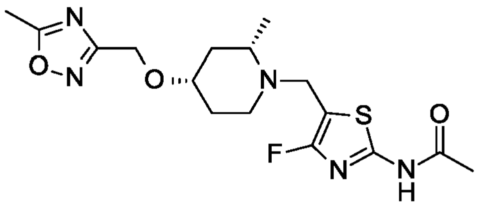

Formula I

,

or a pharmaceutically acceptable salt thereof, wherein the compound, or pharmaceutically acceptable salt thereof is administered orally with a total dose of the compound of 0.1 mg/day to 5 mg/day for use in treating Alzheimer's disease.

In an embodiment, the invention provides a compound of Formula I:

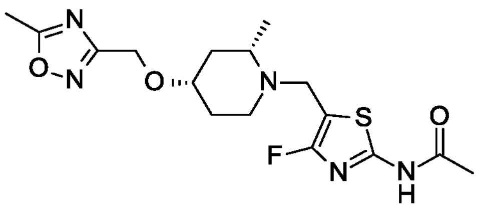

Formula I

,

or a pharmaceutically acceptable salt thereof, wherein the compound, or pharmaceutically acceptable salt thereof is administered orally with a total dose of the compound of 0.1 mg/day to 5 mg/day for use in treating progressive supranuclear palsy.

In an embodiment, the invention provides a compound of Formula I:

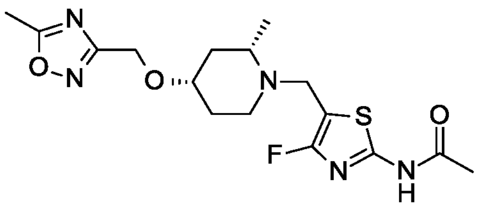

Formula I

,

or a pharmaceutically acceptable salt thereof, wherein the compound, or pharmaceutically acceptable salt thereof is administered orally with a total dose of the compound of 0.1 mg/day to 5 mg/day for use in preventing the progression of mild cognitive impairment to Alzheimer's disease.

In a non-claimed aspect, the disclosure provides the use of a compound of Formula I:

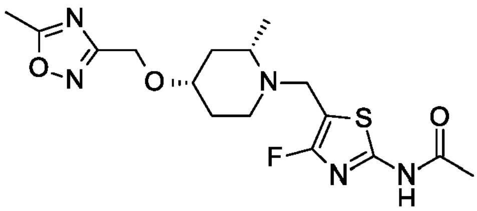

Formula I

or a pharmaceutically acceptable salt thereof, for the manufacture of a medicament for treating a neurodegenerative disease wherein the compound, or pharmaceutically acceptable salt thereof is administered orally with a total dose of the compound of 0.1 mg/day to 5 mg/day.

In a non-claimed aspect, the disclosure provides the use of a compound of Formula I:

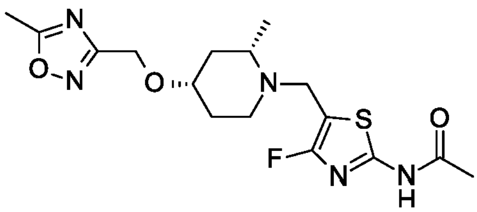

Formula I

or a pharmaceutically acceptable salt thereof, for the manufacture of a medicament for treating Alzheimer's disease wherein the compound, or pharmaceutically acceptable salt thereof is administered orally with a total dose of the compound of 0.1 mg/day to 5 mg/day.

In a non-claimed aspect, the disclosure provides a compound of Formula I:

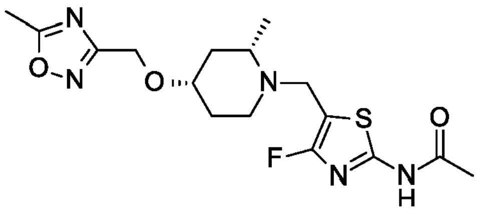

Formula I

or a pharmaceutically acceptable salt thereof, for the manufacture of a medicament for treating progressive supranuclear palsy wherein the compound, or pharmaceutically acceptable salt thereof is administered orally with a total dose of the compound of 0.1 mg/day to 5 mg/day.

In a non-claimed aspect, the disclosure provides the use of a compound of Formula I:

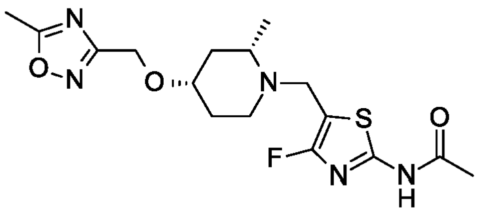

Formula I

or a pharmaceutically acceptable salt thereof, for the manufacture of a medicament for preventing the progression of mild cognitive impairment to Alzheimer's disease, wherein the compound, or pharmaceutically acceptable salt thereof is administered orally with a total dose of the compound of 0.1 mg/day to 5 mg/day.

In a non-claimed aspect, the disclosure provides a method of treating a neurodegenerative disease, comprising administering to a patient in need of such treatment a compound of Formula I:

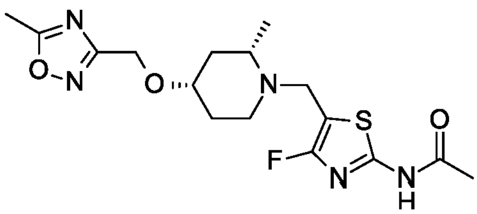

Formula I

or a pharmaceutically acceptable salt thereof, wherein the compound, or pharmaceutically acceptable salt thereof is administered orally with a total dose of of the compound of 0.1 mg/day to 5 mg/day.

In a non-claimed aspect, the disclosure provides a method of treating Alzheimer's disease, comprising administering to a patient in need of such treatment a compound of Formula I:

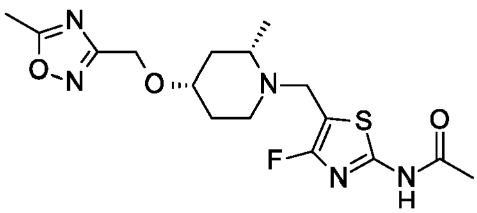

Formula I

or a pharmaceutically acceptable salt thereof, wherein the compound, or pharmaceutically acceptable salt thereof is administered orally with a total dose of the compound of 0.1 mg/day to 5 mg/day.

In a non-claimed aspect, the disclosure provides a method of treating progressive supranuclear palsy, comprising administering to a patient in need of such treatment a compound of Formula I:

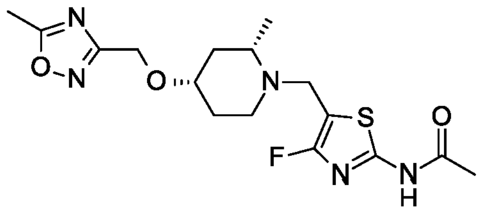

Formula I

or a pharmaceutically acceptable salt thereof, wherein the compound, or pharmaceutically acceptable salt thereof is administered orally with a total dose of the compound of 0.1 mg/day to 5 mg/day.

In a non-claimed aspect, the disclosure provides a method of preventing the progression of mild cognitive impairment to Alzheimer's disease, comprising administering to a patient in need of such treatment a compound of the of Formula I:

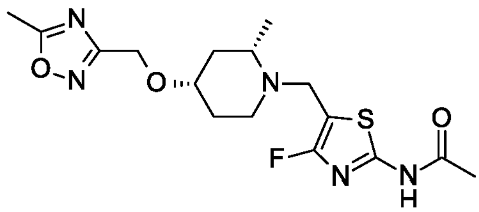

Formula I

or a pharmaceutically acceptable salt thereof, wherein the compound, or pharmaceutically acceptable salt thereof is administered orally with a total dose of the compound of 0.1 mg/day to 5 mg/day.

In an embodiment, the invention provides a pharmaceutical composition, comprising a compound of Formula I:

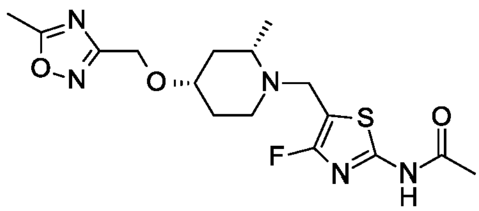

Formula I

,

or a pharmaceutically acceptable salt thereof, for use in treating a neurodegenerative disease wherein the pharmaceutical composition is administered orally with a total dose of the compound of 0.1 mg/day to 5 mg/day.

In an embodiment, the invention provides a pharmaceutical composition for oral administration comprising a compound of Formula I:

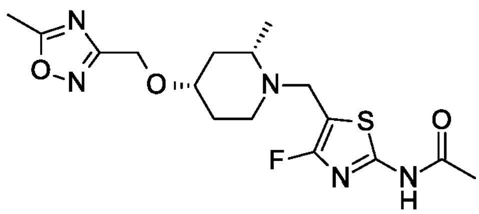

Formula I

or a pharmaceutically acceptable salt thereof with one or more pharmaceutically acceptable carriers, diluents, or excipients wherein the pharmaceutical composition contains a total dose of the compound of 0.1 mg to 5 mg.

In an embodiment, the invention provides a pharmaceutical composition for oral administration comprising a compound of Formula I:

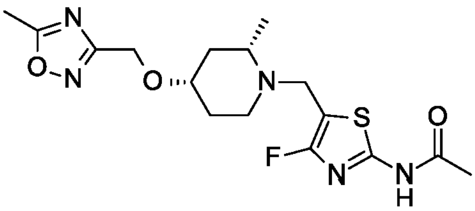

Formula I

or a pharmaceutically acceptable salt thereof with one or more pharmaceutically acceptable carriers, diluents, or excipients wherein the pharmaceutical composition contains a total dose of the compound of 0.1 mg to 5 mg in unit dose form wherein the unit dose is administered once per day.

In an embodiment, the invention provides a pharmaceutical composition for oral administration comprising a compound of Formula I:

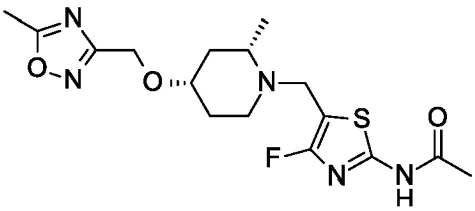

Formula I

or a pharmaceutically acceptable salt thereof with one or more pharmaceutically acceptable carriers, diluents, or excipients wherein the pharmaceutical composition contains a total dose of the compound of 0.05 mg to 2.5 mg in unit dose form wherein the unit dose is administered twice per day.

In an embodiment, it is understood that the compound of Formula I is in the free base form.

In a particular embodiment, the total dose of the compound of Formula I is 0.25 mg/day to 5 mg/day.

In a particular embodiment, the total dose of the compound of Formula I is 0.1 mg/day to 3 mg/day.

In a particular embodiment, the total dose of the compound of Formula I is 0.25 mg/day to 3 mg/day.

In a particular embodiment, the total dose of the compound of Formula I is 0.1 mg/day to 2 mg/day.

In a particular embodiment, the total dose of the compound of Formula I is 0.25 mg/day to 2 mg/day.

In a particular embodiment, the total dose of the compound of Formula I is 0.1 mg/day to 1 mg/day.

In a particular embodiment, the total dose of the compound of Formula I is 0.25 mg/day to 1 mg/day.

In a particular embodiment, the total dose of the compound of Formula I is 5 mg/day.

In a particular embodiment, the total dose of the compound of Formula I is 3 mg/day.

In a particular embodiment, the total dose of the compound of Formula I is 2.5 mg/day.

In a particular embodiment, the total dose of the compound of Formula I is 2 mg/day.

In a particular embodiment, the total dose of the compound of Formula I is 1.5 mg/day.

In a particular embodiment, the total dose of the compound of Formula I is 1 mg/day.

In a particular embodiment, the total dose of the compound of Formula I is 0.75 mg/day.

In a particular embodiment, the total dose of the compound of Formula I is 0.5 mg/day.

In a particular embodiment, the total dose of the compound of Formula I is 0.25 mg/day.

In a particular embodiment, the total dose of the compound of Formula I is 0.1 mg/day.

In a particular embodiment, the compound of Formula I or pharmaceutically acceptable salt thereof is administered in unit dosage form.

In a particular embodiment, the unit dosage form is selected from a capsule, tablet, gelcap, liquid solution, and liquid suspension.

In a particular embodiment, the unit dosage form is a capsule.

In a particular embodiment, the unit dosage form is a tablet.

In a particular embodiment, the total daily dose of the compound of Formula I is administered in one unit dose.

In a particular embodiment, the total daily dose of the compound of Formula I is administered in two unit doses.

In a particular embodiment, the total daily dose of the compound of Formula I is administered in two unit doses each containing equal amounts of the compound of Formula I.

In a particular embodiment, the total daily dose of the compound of Formula I is in two unit doses wherein the administration of each unit dose is separated by at least 8 hours.

In a particular embodiment, the total daily dose of the compound of Formula I is in two unit doses each containing equal amounts of the compound of Formula I wherein the administration of each unit dose is separated by at least 8 hours.

Mild cognitive impairment has been defined as a potential prodromal phase of dementia associated with Alzheimer's disease based on clinical presentation and on progression of patients exhibiting mild cognitive impairment

to Alzheimer's dementia over time. The term "preventing the progression of mild cognitive impairment to Alzheimer's disease" includes restraining, slowing, stopping, or reversing the progression of mild cognitive impairment to Alzheimer's disease in a patient.

As used herein, the terms "treating" or "to treat" includes restraining, slowing, stopping, or reversing the progression or severity of an existing symptom, disease, or disorder.

As used herein, the term "patient" refers to a human.

The compounds of the present invention are formulated as pharmaceutical compositions for oral administration. Such pharmaceutical compositions and processes for preparing same are well known in the art (See, e.g., Remington: The Science and Practice of Pharmacy, L.V. Allen, Editor, 22nd Edition, Pharmaceutical Press, 2012).

The compound of Formula I, or pharmaceutically acceptable salts thereof, may be prepared by a variety of procedures known to one of ordinary skill in the art, for example following the procedures disclosed in United States Patent No. 10,081,625.

The compound of Formula I is also known as N-[4-fluoro-5-[[(2S,4S)-2-methyl-4-[(5-methyl-1,2,4-oxadiazol-3-yl)methoxy]-1-piperidyl]methyl]thiazol-2-yl]acetamide.

<u>A Single Ascending Dose Study in Healthy Subjects to Assess the Safety and Pharmacokinetics of the Compound of Formula I</u>

A Phase 1, single-center, subject- and investigator-blind, single-ascending dose, placebo-controlled, crossover, randomized study is performed to evaluate safety, tolerability, and pharmacokinetics (PK) of the compound of Formula I in healthy subjects. The study is conducted in 2 alternating cohorts (cohorts 1 and 2) in up to 3 study periods across 6 dose levels. Subjects are randomized to 1 of 3 treatment sequences in each cohort, with each sequence including 2 doses of compound of Formula I and 1 placebo dose over the 3 study periods in a complete crossover manner. The clinical study design is summarized in Table 1.

**Table 1. Clinical Study Design**

| | **Cohort 1** | **Cohort 2** |
|---|---|---|
| **Period 1** | 0.15 mg compound of Formula I or placebo | --- |
| **Period 1** | ≥7 days wash-out | 0.6 mg compound of Formula I or placebo |
| **Period 2** | 2 mg compound of Formula I or placebo | ≥ 7 days wash-out |
| **Period 2** | ≥ 7 days wash-out | 5 mg compound of Formula I or placebo |
| **Period 3** | 10 mg compound of Formula I or placebo | ≥ 7 days wash-out |
| **Period 3** | --- | 16 mg compound of Formula I or placebo |

After an overnight fast of at least 8 hours, oral capsules are administered with approximately 240 mL of room-temperature water in the morning of each dosing day in a sitting position. Doses of 0.15 mg to 2 mg are administered as an oral solution of compound of Formula I via an oral dosing syringe with water similar to oral capsule dosing. Tables 2a and 2b summarize the treatment regimens.

**Table 2a. Treatment Regimens for Compound of Formula I.**

| | | |
|---|---|---|
| Dosage Strength (mg) | 0.15 to 2 | 5 to 16 |
| Dosage formulation | Oral solution | Capsule |
| Dosage | Extemporaneous preparation | Extemporaneous preparation |
| Route of administration | Oral | Oral |
| Dosing instructions | Single dose | Single dose |

**Table 2b. Treatment Regimens for Placebo.**

| | | |
|---|---|---|
| Dosage Strength (mg) | Not applicable | Not applicable |
| Dosage formulation | Oral solution (vehicle) | Capsule (HPMC[1]) |
| Dosage | Matching placebo | Matching placebo |

(continued)

| | | |
|---|---|---|
| Dosage Strength (mg) | Not applicable | Not applicable |
| Route of administration | Oral | Oral |
| Dosing instructions | Single dose | Single dose |
| [1] HPMC = hypromellose | | |

The capsules containing the compound of Formula I are prepared extemporaneously. Oral doses of 0.15 mg to 2 mg of the compound of Formula I are prepared extemporaneously as drug in solution. For any specific cohort/dosing period, the total number of capsules administered is the same for all subjects, regardless of whether assigned to placebo or the compound of Formula I. However, the number of capsules may vary between dosing periods and cohorts. This is similarly the case for extemporaneously prepared oral dosing solutions to maintain the blind. The compound of Formula I is supplied in the form of free base with no inactive ingredients for extemporaneous preparation. A matching volume of oral solution vehicle without compound of Formula I is used as placebo for the doses of 0.15 mg to 2 mg.

Venous blood samples of approximately 3 mL each are collected to determine the plasma concentrations of compound of Formula I. Concentrations of compound of Formula I are assayed using a validated liquid chromatography with tandem mass spectrometry method. PK parameter estimates for compound of Formula I are calculated using standard noncompartmental methods of analysis. Plasma concentrations of compound of Formula I are summarized by administered dose of compound of Formula I and the approximate time of PK blood sample collection. Following the procedure essentially as described above, PK data for a single ascending dose of the compound of Formula I in healthy subjects are set forth in Table 3 and Table 4.

**Table 3. Mean plasma concentrations for a single ascending dose study following oral administration of the compound of Formula I in healthy subjects.**

| Dose (mg) Compound of Formula I | 0.15 | 0.6 | 2 | 5 | 10 | 16 |
|---|---|---|---|---|---|---|
| Time (h) | Compound of Formula I Plasma Concentration (ng/mL) | | | | | |
| 0.5 | 0.271 | 6.15 | 16.2 | 14.6 | 28.8 | 59.7 |
| 1 | 0.278 | 6.25 | 21.8 | 58.9 | 122 | 150 |
| 1.5 | 0.246 | 5.21 | 19.5 | 55.3 | 127 | 183 |
| 2 | 0.179 | 4.84 | 18.6 | 50.3 | 125 | 187 |
| 3 | 0.161 | 3.96 | 15.3 | 43.1 | 106 | 153 |
| 4 | ... | 3.6 | 12.6 | 36.5 | 100 | 127 |
| 5 | ... | 3.02 | 10.5 | 29.5 | 81.7 | 100 |
| 6 | ... | 2.35 | 8.75 | 24.8 | 72.3 | 88 |
| 8 | ... | 2.1 | 7.09 | 18.4 | 55.1 | 63 |
| 12 | ... | 1.09 | 4.24 | 9.74 | 32.7 | 32.2 |
| 24 | ... | 0.307 | 1.34 | 2.76 | 11 | 6.83 |
| 48 | ... | ... | ... | 0.634 | 2.28 | 0.828 |

**Table 4. PK parameters of compound of Formula I following oral administration in healthy subjects[1].**

| Dose (mg) [2] | 0.15 mg | 0.6 mg | 2 mg | 5 mg | 10 mg | 16 mg |
|---|---|---|---|---|---|---|
| N[3] | 8 | 7 | 6 | 7 | 6 | 5 |
| CL/F[4] (L/h) | NC[14] | 13.6 (33) | 15.1 (88) | 12.6 (39) | 8.20 (28) | 11.9 (19) |
| AUC(0-∞)[5] (ngxh/mL) | NC[14] | 44.0 (33) | 133 (88) | 396 (39) | 1220 (28) | 1340 (19) |
| Cmax[6] (ng/mL) | 0.318 (52) | 6.61 (30) | 20.9 (38) | 59.1 (18) | 134 (17) | 199 (25) |
| $V_Z/F$[7] (L) | NC[14] | 120 (13) | 119 (40) | 114 (25) | 104 (21) | 102 (23) |

(continued)

| Dose (mg) [2] | 0.15 mg | 0.6 mg | 2 mg | 5 mg | 10 mg | 16 mg |
|---|---|---|---|---|---|---|
| $t_{1/2}$[8] (h) | NC[14] | 6.08 (4.35-9.76) | 5.48 (3.94-11.2) | 6.23 (3.82-8.48) | 8.82 (6.91-12.4) | 5.92 (4.40-7.33) |
| $t_{max}$[9] (h) | 1.02 (0.50-1.50) | 1.00 (0.50-1.50) | 1.25 (1.00-2.00) | 1.02 (1.00-3.00) | 1.50 (1.00-2.07) | 1.98 (1.00-2.00) |
| Ae0-24[10] (mg) | 0.003 (52%)[12] | 0.048 (53%) | 0.155 (57%) | 0.436 (57%) | 1.3 (30%) | 1.14 (36%) |
| CLr[11] (L/h) | NC[14] | 1.17 (30) | 1.25 (30) | 1.18 (32) | 1.22 (10) | 0.96 (46)[13] |

[1]Data presented as geometric mean (%CV geometric mean) unless noted otherwise
[2]Amount of compound of Formula I administered
[3]N = number of subjects
[4]CL/F = apparent clearance calculated after oral administration
[5]AUC(0-∞) = area under the concentration versus time curve from time zero to infinity
[6]Cmax = maximum observed drug concentration
[7]$V_z$/F = apparent volume of distribution after oral administration
[8]$t_{1/2}$ = terminal half-life (geometric mean (min - max))
[9]$t_{max}$ = time of maximum observed drug concentration; median (min - max)
[10]Ae0-24 = amount of compound of Formula I excreted in urine up to 24 hours postdose
[11]CLr = renal clearance
[12]N = 5
[13]N = 3
[14]NC = not calculated

The data discloses that the AUC(0-∞) and $C_{max}$ increase approximately dose-proportionally over the 0.6-mg to 16-mg dose range for the compound of Formula I. The median $t_{max}$ is about 1 hour and $t_{1/2}$ is about 6 hours for the compound of Formula I.

Assessment of Brain O-GlcNAcase Enzyme Occupancy after Single Oral Doses of the Compound of Formula I as Measured by Positron Emission Tomography with the Radioligand [$^{18}$F]LSN3316612 in Healthy Subjects

A single-center, open-label, nonrandomized, positron emission tomography (PET) study demonstrating brain penetration and target engagement of brain O-GlcNAcase (OGA) after single oral doses of 0.25 mg, 1 mg, and 5 mg of N-[4-fluoro-5-[[(2S,4S)-2-methyl-4-[(5-methyl-1,2,4-oxadiazol-3-yl)methoxy]-1-piperidyl]methyl]thiazol-2-yl]acetamide (compound of Formula I) is carried out by one of ordinary skill in the art essentially as set forth below. [$^{18}$F]LSN3316612 is a positron-emitting radiopharmaceutical for *in vivo* imaging of OGA in the brain and is used to evaluate target engagement of compounds which inhibit OGA. The preparation and use of $^{18}$F-LSN3316612 as a PET radioligand is known in the art, for example as described by S. Lu, et. al, Science Translational Medicine, 12, eaau2939 (2020) 13May2020. This single-dose PET study using the [$^{18}$F]LSN3316612 tracer assesses the brain OGA enzyme occupancy (EO) across a suitable range of doses that have been demonstrated to be safe and well tolerated.

Healthy subjects are assigned to 1 of 4 cohorts with 4 subjects in each cohort completing the study. All subjects undergo one baseline PET scan and two post-dose PET scans. A baseline PET scan is performed from up to approximately 14 days before dosing of the compound of Formula I. Overall, each subject receives a single dose of the compound of Formula I and 3 administrations of the [$^{18}$F]LSN3316612 PET tracer. Dosing of the compound of Formula I occurs after completion of the baseline PET scan. Scans are conducted at approximately 2 and 24 hours post-dose for the 0.25 mg and 5 mg doses of the compound of Formula I, and at approximately 2 and 24 hours or 30 and 54 hours post-dose for the 1 mg dose of the compound of Formula I. Dynamic PET data of the brain are acquired over 120 min immediately following tracer injection. EO is summarized by the compound of Formula I dose and approximate scanning time.

The compound of Formula I is administered orally in capsule formulation for doses ≥3 mg. For doses lower than 3 mg, the compound of Formula I is weighed into a suitable container and dissolved in an appropriate volume of degassed Sprite® or diluent.

[$^{18}$F]LSN3316612 is produced in the clinical site radiochemistry facility from the nonradioactive precursor on the day of each PET scan. [$^{18}$F]LSN3316612 injection is a clear solution for intravenous injection formulated in normal saline containing ethanol, sterile water for injection, and sodium ascorbate. [$^{18}$F]LSN3316612 is delivered in normal saline (0.9% NaCl) formulated with the intent to contain approximately 3.3% (v/v) ethanol (EtOH) and sodium ascorbate (4.67 mg/mL).

[$^{18}$F]LSN3316612 is administered intravenously over a 3-minute infusion period using an infusion pump followed by a 10-mL saline flush. Prior to the PET imaging, subjects have an intravenous catheter (for radiotracer infusion) inserted according to standard clinical practice. Each subject receives a single injection of [$^{18}$F]LSN3316612 at each imaging visit. The radiopharmaceutical is injected intravenously at a dose of approximately 5 mCi (not more than 6 mCi), with a maximum mass dose of 10 μg and maximum volume of 10 mL.

For each PET scan, the radiochemistry laboratory synthesizes the radioligand from the precursor according to PET unit production protocol known in the art, such as that described by Lee, J., Liow, J., Paul, S. et al. PET quantification of brain O-GlcNAcase with [18F]LSN3316612 in healthy human volunteers. EJNMMI Res 10, 20 (2020). https://doi.org/10.1186/s13550-020-0616-4.

Arterial blood samples are collected from all subjects during each PET scan to measure radioactivity to provide input for the PET tracer kinetic analysis. Venous blood samples are collected following dosing of compound of Formula I to measure plasma concentrations of compound of Formula I using a validated liquid chromatography with tandem mass spectrometry assay.

The primary imaging outcome for the [$^{18}$F]LSN3316612 PET is the total distribution volume ($V_T$) which is determined in regions where OGA is present, including cortical regions, regions of basal ganglia, thalamus, and cerebellum. Analysis uses the decay-corrected time activity data in the different brain regions. Imaging data is analyzed with 2-tissue compartment model with arterial input function to determine $V_T$. OGA EO after a single dose of the compound of Formula I is obtained using graphical analysis according to the occupancy plot:

$$V_T(\text{Baseline}) - V_T(\text{Dosing}) = \text{Occupancy} * (V_T(\text{Baseline}) - V_{ND}),$$

where VT(Baseline) and VT (Dosing) are the total distribution volumes in several regions obtained at baseline and after compound of Formula I administration, respectively. The occupancy is determined as the slope of the linear regression of the plot, and the nondisplaceable volume of distribution VND as the x-intercept.

Following the procedure essentially as described above, the target engagement of brain OGA after single oral doses of 0.25 mg, 1 mg, and 5 mg of compound of Formula I is set forth in Tables 5a-5c.

**Table 5a: Brain OGA Occupancy for the 0.25 mg dose of Compound of Formula I.**

| Approximate Time (h) | Mean Enzyme Occupancy (%) | Mean Plasma Concentration of Compound of Formula I (ng/mL) |
|---|---|---|
| 0.5 | | 0.934 |
| 1 | | 0.595 |
| 2 | 25.6 | 0.373 |
| 3 | | 0.391 |
| 4 | | 0.367 |
| 5 | | 0.39 |
| 6 | | 0.357 |
| 8 | | 0.294 |
| 12 | | 0.213 |
| 24 | 46 | 0.107 |

**Table 5b: Brain OGA Occupancy for the 1 mg dose of Compound of Formula I.**

| Approximate Time (h) | Mean Enzyme Occupancy (%) | Mean Plasma Concentration of Compound of Formula I (ng/mL) |
|---|---|---|
| 0.5 | | 6.29 |
| 1 | | 6.24 |
| 2 | 97.3 | 5.75 |
| 3 | | 4.97 |
| 4 | | 4.28 |

(continued)

| Approximate Time (h) | Mean Enzyme Occupancy (%) | Mean Plasma Concentration of Compound of Formula I (ng/mL) |
|---|---|---|
| 5 | | 3.87 |
| 6 | | 3.08 |
| 8 | | 2.45 |
| 12 | | 1.42 |
| 24 | 80.6 | 0.412 |
| 30 | 68 | 0.222 |
| 54 | 30.3 | 0.0735 |

**Table 5c: Brain OGA Occupancy for the 5 mg dose of Compound of Formula I.**

| Approximate Time (h) | Mean Enzyme Occupancy (%) | Mean Plasma Concentration of Compound of Formula I (ng/mL) |
|---|---|---|
| 0.5 | | 32.4 |
| 1 | | 30.7 |
| 2 | 98.2 | 36 |
| 3 | | 31.9 |
| 4 | | 31.5 |
| 5 | | 30.6 |
| 6 | | 25.3 |
| 8 | | 17.8 |
| 12 | | 12.5 |
| 24 | 92.5 | 3.07 |

The data provided in Tables 5a-5c above discloses a plasma concentration-dependent change in brain OGA EO with EO for the 5 mg dose of the compound of Formula I exceeding 90% EO at 24 hours post dose. The 1 mg dose of the compound of Formula I was found to be 80.6% EO at 24 hours post dose and 30.3% EO at 54 hours post dose. The 0.25 mg dose of the compound of Formula I was found to be 46% EO at 24 hours post dose.

Utilizing the PK data from the single ascending dose study of the compound of Formula I in healthy subjects and the PET study demonstrating brain penetration and target engagement of brain OGA after single oral doses of the compound of Formula I as disclosed above, the low doses and dosage regimens for treating a neurodegenerative disease, including AD, PSP, FTD, CBS, and other neurodegenerative tauopathies, with a compound of Formula I are set forth below:

A total dose of the compound of Formula I of 0.25 mg/day to 5 mg/day.
A total dose of the compound of Formula I of 0.1 mg/day to 3 mg/day.
A total dose of the compound of Formula I of 0.25 mg/day to 3 mg/day.
A total dose of the compound of Formula I of 0.1 mg/day to 2 mg/day.
A total dose of the compound of Formula I of 0.25 mg/day to 2 mg/day.
A total dose of the compound of Formula I of 0.1 mg/day to 1 mg/day.
A total dose of the compound of Formula I of 0.25 mg/day to 1 mg/day.
A total dose of the compound of Formula I of 5 mg/day.
A total dose of the compound of Formula I of 3 mg/day.
A total dose of the compound of Formula I of 2.5 mg/day.
A total dose of the compound of Formula I of 2 mg/day.
A total dose of the compound of Formula I of 1.5 mg/day.
A total dose of the compound of Formula I of 1 mg/day.
A total dose of the compound of Formula I of 0.75 mg/day.

A total dose of the compound of Formula I of 0.5 mg/day.
A total dose is the compound of Formula I of 0.25 mg/day.
A total dose of the compound of Formula I of 0.1 mg/day.

It is preferred that the total daily dose of compound of Formula I administered is in one unit dose.

It is further preferred that the total daily dose of the compound of Formula I administered is in two unit doses. It is preferred that the total daily dose of the compound of Formula I is administered in two unit doses wherein each dose contains equal amounts of the compound of Formula I. It is preferred that when the total daily dose of the compound of Formula I is administered in two unit doses, the administration of each unit dose is separated by at least 8 hours.

In addition, total doses of the compound of Formula I in a pharmaceutical composition are set forth below:

A total dose of the compound of Formula I is 0.25 mg to 5 mg.
A total dose of the compound of Formula I is 0.1 mg to 3 mg.
A total dose of the compound of Formula I is 0.25 mg to 3 mg.
A total dose of the compound of Formula I is 0.1 mg to 2 mg.
A total dose of the compound of Formula I is 0.25 mg to 2 mg.
A total dose of the compound of Formula I is 0.1 mg to 1 mg.
A total dose of the compound of Formula I is 0.25 mg to 1 mg.
A total dose of the compound of Formula I is 3 mg.
A total dose of the compound of Formula I of 2.5 mg/day.
A total dose of the compound of Formula I is 2 mg.
A total dose of the compound of Formula I is 1.5 mg.
A total dose of the compound of Formula I is 1 mg.
A total dose of the compound of Formula I is 0.75 mg.
A total dose of the compound of Formula I of 0.5 mg/day.
A total dose of the compound of Formula I is 0.25 mg.

In addition, it is preferred that the total dose of the pharmaceutical composition comprising the compound of Formula I, or pharmaceutically acceptable salt thereof is contained in one unit dose.

It is further preferred that the total dose of the pharmaceutical composition comprising the compound of Formula I, or pharmaceutically acceptable salt thereof is contained in one unit dose wherein the single unit dose is administered once per day.

It is also preferred that the total dose of the pharmaceutical composition comprising the compound of Formula I, or pharmaceutically acceptable salt thereof is contained in two unit doses.

It is preferred that the total dose of the pharmaceutical composition comprising the compound of Formula I, or pharmaceutically acceptable salt thereof is contained in two unit doses with each unit dose containing equal amounts of the compound of Formula I.

It is further preferred that the total dose of the pharmaceutical composition comprising the compound of Formula I, or pharmaceutically acceptable salt thereof is contained in two unit doses wherein each dose is administered in one day.

It is further preferred that the total dose of the pharmaceutical composition comprising the compound of Formula I, or pharmaceutically acceptable salt thereof is contained in two unit doses with each unit dose containing equal amounts of the compound of Formula I wherein each dose is administered within one day, preferably separated by at least 8 hours.

## Claims

**1.** A compound of the formula:

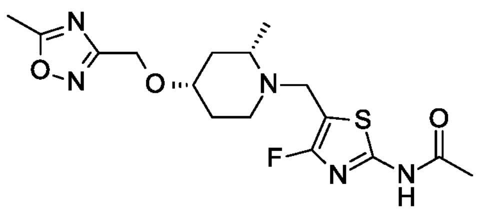

,

or a pharmaceutically acceptable salt thereof, for use in treating a neurodegenerative disease wherein the compound,

or pharmaceutically acceptable salt thereof is administered orally with a total dose of the compound of 0.1 mg/day to 5 mg/day.

2. The compound, or pharmaceutically acceptable salt thereof for use according to claim 1 wherein the neurodegenerative disease is Alzheimer's disease.

3. The compound, or pharmaceutically acceptable salt thereof for use according to claim 1 wherein the neurodegenerative disease is progressive supranuclear palsy.

4. A compound of the formula:

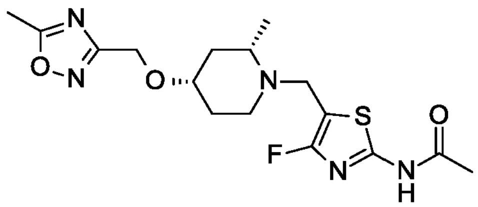

,

or a pharmaceutically acceptable salt thereof, for use in preventing the progression of mild cognitive impairment to Alzheimer's disease wherein the compound, or pharmaceutically acceptable salt thereof is administered orally with a total dose of the compound of 0.1 mg/day to 5 mg/day.

5. The compound, or pharmaceutically acceptable salt thereof for use according to any one of claims 1 to 4 wherein the total dose of the compound is selected from 0.25 mg/day to 5 mg/day, 0.1 mg/day to 3 mg/day, 0.25 mg/day to 3 mg/day, 0.1 mg/day to 2 mg/day, 0.25 mg/day to 2 mg/day, 0.1 mg/day to 1 mg/day, and 0.25 mg/day to 1 mg/day.

6. The compound, or pharmaceutically acceptable salt thereof for use according to any one of claims 1 to 4 wherein the total dose of the compound is selected from 3 mg/day, 2.5 mg/day, 2 mg/day, 1.5 mg/day, 1 mg/day, 0.75 mg/day, 0.5 mg/day, and 0.25 mg/day.

7. The compound, or pharmaceutically acceptable salt thereof for use according to any one of claims 1 to 6 wherein the compound or pharmaceutically acceptable salt thereof is administered in unit dosage form.

8. The compound, or pharmaceutically acceptable salt thereof for use according to claim 7 wherein the unit dosage form is selected from a capsule, tablet, gelcap, liquid solution, and liquid suspension.

9. The compound, or pharmaceutically acceptable salt thereof for use according to claim 8 wherein the unit dosage form is a capsule.

10. The compound, or pharmaceutically acceptable salt thereof for use according to claim 8 wherein the unit dosage form is a tablet.

11. The compound, or pharmaceutically acceptable salt thereof for use according to any one of claims 7 to 10 wherein the total daily dose is administered in one unit dose.

12. The compound, or pharmaceutically acceptable salt thereof for use according to any one of claims 7 to 10 wherein the total daily dose is administered in two unit doses.

13. The compound, or pharmaceutically acceptable salt thereof for use according to claim 12 wherein the administration of each unit dose is separated by at least 8 hours.

14. A pharmaceutical composition for oral administration, comprising a compound of the formula:

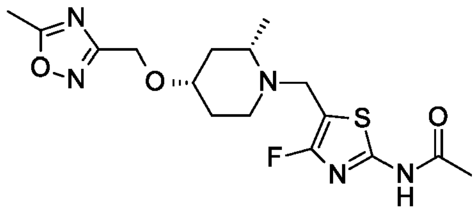

,

or a pharmaceutically acceptable salt thereof with one or more pharmaceutically acceptable carriers, diluents, or excipients wherein the pharmaceutical composition contains a total dose of the compound of 0.1 mg to 5 mg.

**15.** The pharmaceutical composition according to claim 14 wherein the total dose of the compound is selected from 0.25 mg to 5 mg, 0.1 mg to 3 mg, 0.25 mg to 3 mg, 0.1 mg to 2 mg, 0.25 mg to 2 mg, 0.1 mg to 1 mg, and 0.25 mg to 1 mg.

**16.** The pharmaceutical composition according to claim 14 wherein the total dose of the compound is selected from 3 mg, 2.5 mg, 2 mg, 1.5 mg, 1 mg, 0.75 mg, 0.5 mg, and 0.25 mg.

**17.** The pharmaceutical composition according to any one of claims 14 to 16 wherein the total dose of the compound is contained in one unit dose.

**18.** The pharmaceutical composition according to any one of claims 14 to 16 wherein the total dose of the compound is contained in two unit doses.

**19.** The pharmaceutical composition according to any one of claims 14 to 16 wherein the total dose of the compound is contained in two unit doses with each unit dose containing equal amounts of the compound.

**Patentansprüche**

**1.** Verbindung der Formel:

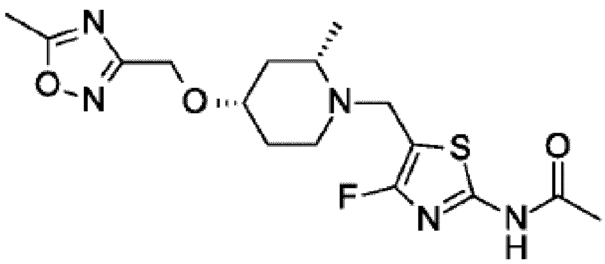

,

oder ein pharmazeutisch verträgliches Salz davon zur Verwendung bei einem Behandeln einer neurodegenerativen Krankheit, wobei die Verbindung oder das pharmazeutisch verträgliche Salz davon mit einer Gesamtdosis der Verbindung von 0,1 mg/Tag bis 5 mg/Tag oral verabreicht wird.

**2.** Verbindung oder pharmazeutisch verträgliches Salz davon zur Verwendung nach Anspruch 1, wobei die neurodegenerative Krankheit eine Alzheimer-Krankheit ist.

**3.** Verbindung oder pharmazeutisch verträgliches Salz davon zur Verwendung nach Anspruch 1, wobei die neurodegenerative Krankheit progressive supranukleäre Blickparese ist.

**4.** Verbindung der Formel:

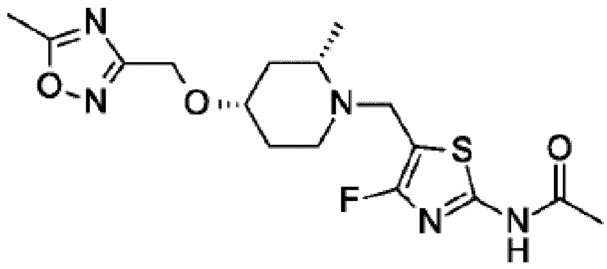

,

oder ein pharmazeutisch verträgliches Salz davon zur Verwendung bei einem Verhindern des Fortschreitens einer leichten kognitiven Beeinträchtigung hin zu der Alzheimer-Krankheit, wobei die Verbindung oder das pharmazeutisch verträgliche Salz davon mit einer Gesamtdosis der Verbindung von 0,1 mg/Tag bis 5 mg/Tag oral verabreicht wird.

**5.** Verbindung oder pharmazeutisch verträgliches Salz davon zur Verwendung nach einem der Ansprüche 1 bis 4, wobei die Gesamtdosis der Verbindung aus 0,25 mg/Tag bis 5 mg/Tag, 0,1 mg/Tag bis 3 mg/Tag, 0,25 mg/Tag bis 3 mg/Tag, 0,1 mg/Tag bis 2 mg/Tag, 0,25 mg/Tag bis 2 mg/Tag, 0,1 mg/Tag bis 1 mg/Tag und 0,25 mg/Tag bis 1 mg/Tag ausgewählt ist.

**6.** Verbindung oder pharmazeutisch verträgliches Salz davon zur Verwendung nach einem der Ansprüche 1 bis 4, wobei die Gesamtdosis der Verbindung aus 3 mg/Tag, 2,5 mg/Tag, 2 mg/Tag, 1,5 mg/Tag, 1 mg/Tag, 0,75 mg/Tag, 0,5 mg/Tag und 0,25 mg/Tag ausgewählt ist.

**7.** Verbindung oder pharmazeutisch verträgliches Salz davon zur Verwendung nach einem der Ansprüche 1 bis 6, wobei die Verbindung oder das pharmazeutisch verträgliche Salz davon in Dosierungseinheitsform verabreicht wird.

**8.** Verbindung oder pharmazeutisch verträgliches Salz davon zur Verwendung nach Anspruch 7, wobei die Dosierungseinheitsform aus einer Kapsel, Tablette, Gelkapsel, flüssigen Lösung und flüssigen Suspension ausgewählt ist.

**9.** Verbindung oder pharmazeutisch verträgliches Salz davon zur Verwendung nach Anspruch 8, wobei die Dosierungseinheitsform eine Kapsel ist.

**10.** Verbindung oder pharmazeutisch verträgliches Salz davon zur Verwendung nach Anspruch 8, wobei die Dosierungseinheitsform eine Tablette ist.

**11.** Verbindung oder pharmazeutisch verträgliches Salz davon zur Verwendung nach einem der Ansprüche 7 bis 10, wobei die tägliche Gesamtdosis in einer Einheitsdosis verabreicht wird.

**12.** Verbindung oder pharmazeutisch verträgliches Salz davon zur Verwendung nach einem der Ansprüche 7 bis 10, wobei die tägliche Gesamtdosis in zwei Einheitsdosen verabreicht wird.

**13.** Verbindung oder pharmazeutisch verträgliches Salz davon zur Verwendung nach Anspruch 12, wobei zwischen der Verabreichung jeder Einheitsdosis ein Abstand von mindestens 8 Stunden liegt.

**14.** Pharmazeutische Zusammensetzung für eine orale Verabreichung, umfassend eine Verbindung der Formel:

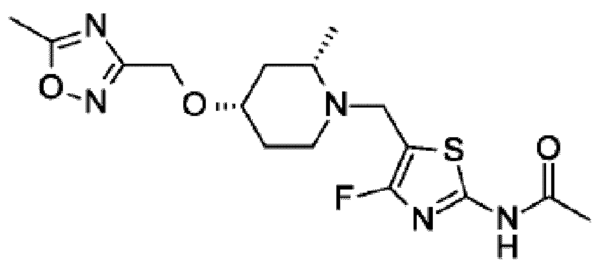

,

oder ein pharmazeutisch verträgliches Salz davon mit einem oder mehreren pharmazeutisch verträglichen Trägern,

Verdünnungsmitteln oder Arzneistoffträgern, wobei die pharmazeutische Zusammensetzung eine Gesamtdosis der Verbindung von 0,1 mg bis 5 mg enthält.

**15.** Pharmazeutische Zusammensetzung nach Anspruch 14, wobei die Gesamtdosis der Verbindung aus 0,25 mg bis 5 mg, 0,1 mg bis 3 mg, 0,25 mg bis 3 mg, 0,1 mg bis 2 mg, 0,25 mg bis 2 mg, 0,1 mg bis 1 mg und 0,25 mg bis 1 mg ausgewählt ist.

**16.** Pharmazeutische Zusammensetzung nach Anspruch 14, wobei die Gesamtdosis der Verbindung aus 3 mg, 2,5 mg, 2 mg, 1,5 mg, 1 mg, 0,75 mg, 0,5 mg und 0,25 mg ausgewählt ist.

**17.** Pharmazeutische Zusammensetzung nach einem der Ansprüche 14 bis 16, wobei die Gesamtdosis der Verbindung in einer Einheitsdosis enthalten ist.

**18.** Pharmazeutische Zusammensetzung nach einem der Ansprüche 14 bis 16, wobei die Gesamtdosis der Verbindung in zwei Einheitsdosen enthalten ist.

**19.** Pharmazeutische Zusammensetzung nach einem der Ansprüche 14 bis 16, wobei die Gesamtdosis der Verbindung in zwei Einheitsdosen enthalten ist, wobei jede Einheitsdosis gleiche Mengen der Verbindung enthält.

## Revendications

**1.** Composé de la formule :

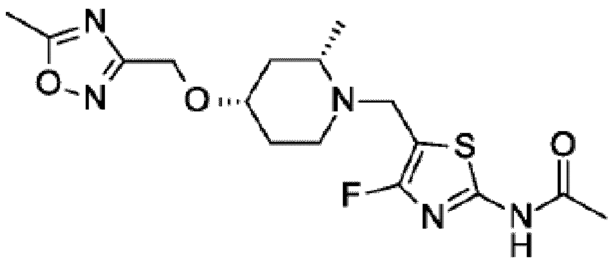

,

ou sel pharmaceutiquement acceptable de celui-ci, pour utilisation dans le traitement d'une maladie neurodégénérative, dans lequel le composé ou le sel pharmaceutiquement acceptable de celui-ci est administré par voie orale à raison d'une dose totale du composé allant de 0,1 mg/jour à 5 mg/jour.

**2.** Composé, ou sel pharmaceutiquement acceptable de celui-ci, pour utilisation selon la revendication 1, dans lequel la maladie neurodégénérative est la maladie d'Alzheimer.

**3.** Composé, ou sel pharmaceutiquement acceptable de celui-ci, pour utilisation selon la revendication 1, dans lequel la maladie neurodégénérative est la paralysie supranucléaire progressive.

**4.** Composé de la formule :

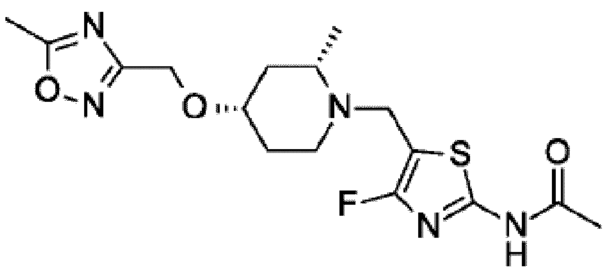

,

ou sel pharmaceutiquement acceptable de celui-ci, pour utilisation dans la prévention de la progression de la

déficience cognitive légère vers la maladie d'Alzheimer, dans lequel le composé ou le sel pharmaceutiquement acceptable de celui-ci est administré par voie orale avec une dose totale du composé allant de 0,1 mg/jour et 5 mg/jour.

5. Composé, ou sel pharmaceutiquement acceptable de celui-ci, pour utilisation selon l'une quelconque des revendications 1 à 4, dans lequel la dose totale du composé est choisie entre 0,25 mg/jour et 5 mg/jour, 0,1 mg/jour et 3 mg/jour, 0,25 mg/jour et 3 mg/jour, 0,1 mg/jour et 2 mg/jour, 0,25 mg/jour et 2 mg/jour, 0,1 mg/jour et 1 mg/jour, et 0,25 mg/jour et 1 mg/jour.

6. Composé, ou sel pharmaceutiquement acceptable de celui-ci, pour utilisation selon l'une quelconque des revendications 1 à 4, dans lequel la dose totale du composé est choisie parmi 3 mg/jour, 2,5 mg/jour, 2 mg/jour, 1,5 mg/jour, 1 mg/jour, 0,75 mg/jour, 0,5 mg/jour et 0,25 mg/jour.

7. Composé, ou sel pharmaceutiquement acceptable de celui-ci, pour utilisation selon l'une quelconque des revendications 1 à 6, dans lequel le composé ou le sel pharmaceutiquement acceptable de celui-ci est administré sous forme de dose unitaire.

8. Composé, ou sel pharmaceutiquement acceptable de celui-ci, pour utilisation selon la revendication 7, dans lequel la forme de dosage unitaire est choisie parmi une capsule, un comprimé, une gélule, une solution liquide et une suspension liquide.

9. Composé, ou sel pharmaceutiquement acceptable de celui-ci, pour utilisation selon la revendication 8, dans lequel la forme de dosage unitaire est une capsule.

10. Composé, ou sel pharmaceutiquement acceptable de celui-ci, pour utilisation selon la revendication 8, dans lequel la forme de dosage unitaire est un comprimé.

11. Composé, ou sel pharmaceutiquement acceptable de celui-ci, pour utilisation selon l'une quelconque des revendications 7 à 10, dans lequel la dose journalière totale est administrée en une seule unité.

12. Composé, ou sel pharmaceutiquement acceptable de celui-ci, pour utilisation selon l'une quelconque des revendications 7 à 10, dans lequel la dose journalière totale est administrée en deux doses unitaires.

13. Composé, ou sel pharmaceutiquement acceptable de celui-ci, pour utilisation selon la revendication 12, dans lequel l'administration de chaque dose unitaire est séparée d'au moins 8 heures.

14. Composition pharmaceutique pour administration orale, comprenant un composé de la formule :

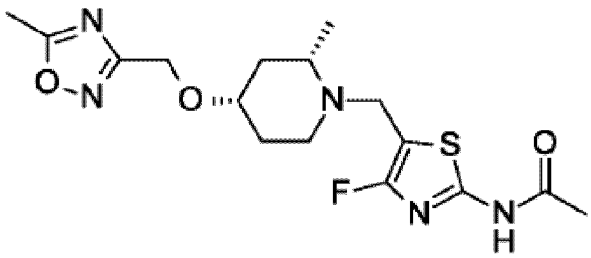

,

ou sel pharmaceutiquement acceptable de celui-ci avec un ou plusieurs supports, diluants ou excipients pharmaceutiquement acceptables, dans laquelle la composition pharmaceutique contient une dose totale du composé allant de 0,1 mg à 5 mg.

15. Composition pharmaceutique selon la revendication 14, dans laquelle la dose totale du composé est choisie entre 0,25 mg et 5 mg, 0,1 mg et 3 mg, 0,25 mg et 3 mg, 0,1 mg et 2 mg, 0,25 mg et 2 mg, 0,1 mg et 1 mg, et 0,25 mg et 1 mg.

16. Composition pharmaceutique selon la revendication 14, dans laquelle la dose totale du composé est choisie parmi 3 mg, 2,5 mg, 2 mg, 1,5 mg, 1 mg, 0,75 mg, 0,5 mg et 0,25 mg.

17. Composition pharmaceutique selon l'une quelconque des revendications 14 à 16, dans laquelle la dose totale du

composé est contenue dans une dose unitaire.

**18.** Composition pharmaceutique selon l'une quelconque des revendications 14 à 16, dans laquelle la dose totale du composé est contenue dans deux doses unitaires.

**19.** Composition pharmaceutique selon l'une quelconque des revendications 14 à 16, dans laquelle la dose totale du composé est contenue dans deux doses unitaires, chaque dose unitaire contenant des quantités égales du composé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10081625 B


### Non-patent literature cited in the description


- **W. COOK**. *Society of Toxicology Annual Meeting (virtual)*, 12 May 2020, https://www.toxicology.org/events/am/AM2020/docs/2020-ePoster-index.pdf
- Remington: The Science and Practice of Pharmacy. Pharmaceutical Press, 2012
- **S. LU**. *Science Translational Medicine*, 13 May 2020, vol. 12, eaau2939
- **LEE, J.** ; **LIOW, J.** ; **PAUL, S. et al.** PET quantification of brain O-GlcNAcase with [18F]LSN3316612 in healthy human volunteers. *EJNMMI Res*, 2020, vol. 10, 20, https://doi.org/10.1186/s13550-020-0616-4